# EUROPEAN PATENT APPLICATION

(11) **EP 1 527 765 A1**
(43) Date of publication of application: **04.05.2005**
(21) Application number: 03405768.7
(22) Date of filing: 27.10.2003
(51) Int. Cl.: A61J 1/00, A61M 35/00, B65D 75/32

(54) **Single dose pack for curative substances for ingestion or contact**

(71) Applicant: Fabbri, Doriano, 6596 Gordola (CH); Minacore, Pietra, 6596 Gordola (CH)
(72) Inventor: Fabbri, Doriano, 6596 Gordola (CH); Minacore, Pietra, 6596 Gordola (CH)
(74) Representative: Fiammenghi-Domenighetti, Delfina

(57) **Abstract**

A single dose pack (1) of substances (2) capable of performing curative and/or soothing functions through ingestion or contact with injured parts, comprising a sealed flexible container (3) of plastics material defining within it a cavity (3c) housing a single dose of the said substances (2) in the liquid state which when converted to the solid state by freezing adopts the shape of the container and is capable of being manipulated in order to reach the said injured parts is described. In the single dose pack in question an osmotic membrane (5) is applied to the inner surface of the said container (3) so as to form a closed envelope which contains within it the said substances (2) when in the solid state, and remains adhering thereto when the container (3) is removed.

## Description

This invention relates to the medical/pharmaceutical sector, and in particular it relates to the sector of the manufacture and use of curative substances which perform their function through ingestion or contact with the injured and/or inflamed parts.
More specifically the invention relates to an innovative single dose pack for curative and/or soothing substances of a type mentioned above in the liquid state.

The invention has been conceived by operators in the hospital sector who have found that, especially in infected or inflammatory conditions of the oropharyngeal cavity and during corresponding postoperative periods or conditions affecting other parts of the body cured by transdermal means, the means currently used to sooth the pain and exert a disinfectant and vasoconstrictive effect have limitations and/or disadvantages.

Particularly in the cases mentioned above, experience has demonstrated that the application of ice to injured, inflamed or bruised parts can alleviate pain. However, the ice is usually applied to the oropharyngeal cavity by gargling, which not all patients are in a position to put into practice, or through invasive means which create appreciable discomfort for the patients without however exerting any specific disinfecting action.

In order to overcome the abovementioned disadvantages single dose packs comprising a container in which a curative substance in the liquid state is located in a cavity thereof of elongated shape have been conceived. Through freezing the said curative substance adopts the shape of a bar of ice in the solid state and through tearing the pack container in question it is possible to manipulate it easily to apply one end thereof to the injured parts, in such a way that an antiseptic action is exerted upon these accompanied by the analgesic and anti-haemorrhagic effect provided by the ice.
An example of such single dose packs is described in US-A - 6 152 468 (Med. Tech. Industries).

Use of the single dose packs mentioned above may however give rise to problems: first of all, for example for applications in the oropharyngeal cavity, the entire volume of the substances contained in the single dose pack, dissolving through heat, comes into contact not only with the parts which require to be cured but also with the mucosa of the tongue and palate, with the additional disadvantage that these substances are swallowed by the patient or have to be expelled by the latter cyclically, in a sequence of operations which cannot always be performed and which is in any event troublesome.
Secondly, depending upon the nature of the aforesaid substances, which mostly consist of a solute dissolved in a solvent, one of these two components may have allergenic and/or inflammatory effects which could reduce or even cancel out the beneficial effects sought.

In order to avoid all the abovementioned disadvantages, the applicants have conceived a new type of single dose pack similar in composition to those already in existence but in which an osmotic membrane is located on the inside surface of the abovementioned container to form a closed envelope which contains the said curative substances within it, adhering thereto, when converted into the solid state, and it also remains bound thereto when the container is removed.
With the single dose pack according to the invention, by taking advantage of the known properties of osmotic membranes and their semipermeability it is possible to ensure that only the solute or the solvent alone can by passing through the pores thereof, reaching the injured part to which the curative substances have to be applied.

By suitably selecting the type of material comprising the osmotic membranes used and/or the mean diameter of their pores it is possible to use a single dose pack according to the invention for multiple curative purposes.
In fact by varying the nature of the curative substances and associating them with a suitably chosen osmotic membrane it is possible to apply active ingredients capable of performing analgesic, disinfectant, cicatrizing, anti-inflammatory, etc., functions from time to time.
The object of this invention therefore comprises a single dose pack for substances capable of performing curative functions as described in appended Claim 1.

Some embodiments of a single dose pack according to the invention will now be described in greater detail making reference to the appended drawings in which:
- Figure 1 is a perspective view of a preferred embodiment of the single dose pack according to the invention,
- Figure 2 is a perspective view of the single dose pack in Figure 1 with the container torn and partly removed with a view to application to the parts requiring treatment,
- Figure 3 is a longitudinal cross-section of the extremity of the frozen body containing the curative substances of the single dose pack in Figures 1, 2 showing the position of the osmotic membrane adhering thereto.

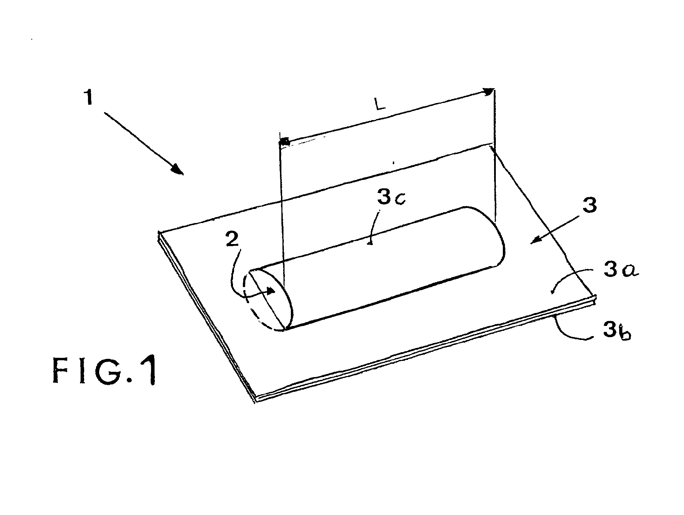

If Figure 1 is considered, it will be seen that a single dose pack 1 according to the invention may essentially comprise a container 3 of flat extent formed by two overlapping sheets 3a, 3b of flexible plastics material.
The two sheets 3a, 3b are attached together by known means, for example by thermal welding, after they have been deformed, again by means known to those skilled in the art, in such a way that when alongside each other they form a cavity 3c of elongated shape designed to receive within it curative substances 2 when they are in the liquid state. In the instance illustrated, aforesaid cavity 3c has an elongated cylindrical shape but its transverse cross-section may be not circular but elliptical, quadrangular, etc.

It is important that this cavity 3c should have a predetermined length L, for example 12 ÷ 15 cm, the purposes of which will be explained below.

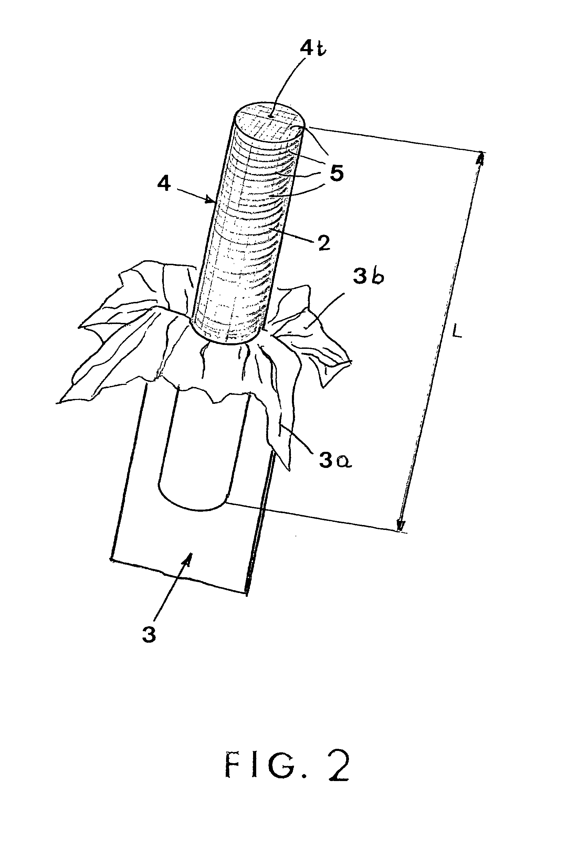

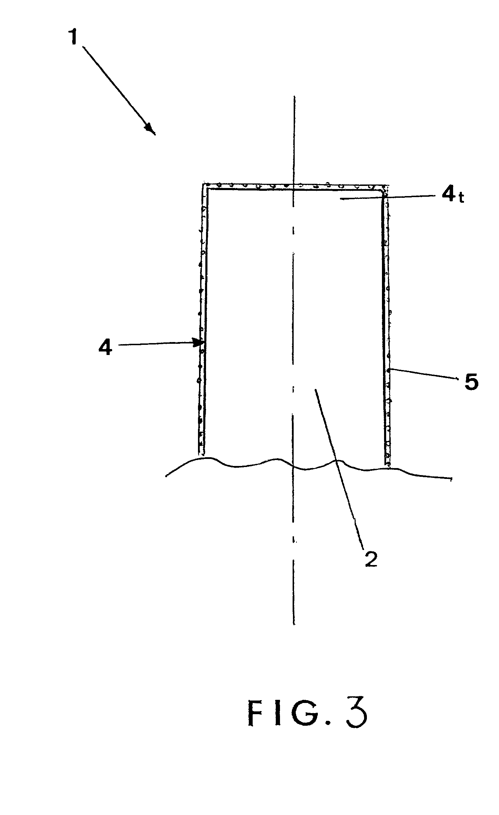

If said substances 2 are converted from the liquid state to the solid state by freezing, they form a bar 4 (see in this respect Figure 2) of a shape which in this case is cylindrical, and if container 3 is torn or cut as illustrated in Figure 2, what is left of container 3 can be seized in the hand, readily manipulating it so as to apply its free end 4t to the injured or inflamed parts so that in this way it can exert the desired soothing and/or curative action upon them.
Figure 2 also illustrates an osmotic membrane 5, which in the construction of a single dose pack 1 is applied so as to adhere to the inner wall of container 3, adhering perfectly to the solid body generated by the freezing of curative liquid substances 2, performing its semi-permeable barrier function as already described previously.
This osmotic membrane 5 which adheres to frozen curative substances 2 even when container 3 is wholly or partly removed can be better seen in Figure 3.

In order to manufacture osmotic membrane 5, which must form a closed envelope containing curative substances 2, the inventors suggest that a material selected from the group comprising cellulose nitrate (CN), cellulose ester (CE), regenerated cellulose (RC), cellulose acetate (CA) and nylon (NY) or a material selected from the group comprising polycarbonate (PC), polyether sulphone (PES), Teflon@ (PTFE), polyvinylidone fluoride (PVDF) should be used.

As regards the diameter of the holes in osmotic membrane 5, the inventors suggest that values between 100 Da and 300,000 Da should be used.
In cases where a single dose pack according to the invention must essentially perform the soothing and antihaemorrhagic function of ice, the inventors have also selected the composition of a number of essentially plant-based infusions.

In a first embodiment the said substances 2 in the liquid state comprise a mixture of the following infusions:
- infusion of plantain for approximately 25% of the weight,
- infusion of mallow for approximately 25% of the weight,
- infusion of camomile for approximately 25% of the weight,
- infusion of sage for approximately 12.5% of the weight,
- infusion of thyme for approximately 6.25% of the weight,
- with the addition of lemon juice for approximately 6.25% of the weight.

In another embodiment in which a better flavour is sought for substances 2, the latter comprise a mixture of the following infusions:
- infusion of plantain for approximately 20% of the weight,
- infusion of mallow for approximately 20% of the weight,
- infusion of camomile for approximately 20% of the weight,
- infusion of sage for approximately 10% of the weight,
- infusion of dog rose with hibiscus flowers for approximately 10% of the weight,
- infusion of orange flowers for approximately 10% of the weight,
- infusion of thyme for approximately 5% of the weight,
- with the addition of lemon juice for approximately 5% of the weight.

When, as already mentioned, a sweetener is added, the inventors suggest that honey should be used in order to obtain a mixture of perfectly natural substances.

It is also pointed out that in order to prepare infusions of the various herbs listed above, which are to be used in a mixture together, the inventors suggest that water (which may be distilled water) in which the following respectively have been infused should be used:
- approximately 1.5% by weight of plantain,
- approximately 0.9% by weight of mallow,
- approximately 0.9% by weight of camomile,
- approximately 1.5% by weight of sage,
- approximately 5.4% by weight of dog rose with hibiscus flowers,
- approximately 2.4% by weight of orange flowers,
- approximately 1% by weight of thyme.

Obviously, depending upon the therapeutic outcomes desired, the composition of the mixtures described above may be varied, and other types of infusion may also be added or the relative percentages of the different infusions may be altered.

It is further disclosed that the inventors are of the opinion that in order to obtain a substantial bar 4 of appropriate dimensions it is sufficient that the said cavity 3c in envelope 3 should have a volume of approximately 30 cm³.

Finally it is pointed out that in a single dose pack according to the invention the type and nature of the container may be modified according to individual requirements - whatever the shape adopted by the bar or body of frozen curative substances, the osmotic membrane, which may be in a reinforced and/or partly rigidified form known to those skilled in the art, may in any event be designed and constructed so as to adhere perfectly, performing its well-known semi-permeable barrier function.

## Claims

1. Single dose pack (1) of substances (2) capable of performing curative and/or soothing functions through ingestion or contact with injured parts, comprising a sealed flexible container (3) of plastics material defining within it a cavity (3c) housing a single dose of the said substances (2) in the liquid state which when converted to the solid state by freezing adopts the shape of the container and is capable of being manipulated in order to reach the said injured parts, **characterized in that** an osmotic membrane (5) is applied to the inner surface of the said container (3) so as to form a closed envelope which contains within it the said substances (2) when in the solid state, and remains adhering thereto when the container (3) is removed.

2. Single dose pack according to Claim 1, in which the holes in the said osmotic membrane (5) have a diameter of between 100 Da and 300,000 Da.

3. Single dose pack according to Claim 2, in which the said osmotic membrane (5) is constructed from a material selected from the group comprising cellulose nitrate (CN), cellulose ester (CE), regenerated cellulose (RC), cellulose acetate (CA) and nylon (NY).

4. Single dose pack according to Claim 2, in which the said osmotic membrane is made of a material selected from the group comprising polycarbonate (PC), polyether sulphone (PES), Teflon@ (PTFE), polyvinylidone fluoride (PVDF).

5. Single dose pack according to one of the preceding claims, in which the said substances (2) in the liquid state comprise a mixture of infusions obtained from the following herbs:
- plantain,
- mallow,
- camomile,
- sage,
- thyme,
to which lemon juice is added.

6. Single dose pack according to Claim 5, in which the said substances (2) in the liquid state comprise a mixture of the following infusions:
- infusion of plantain for approximately 25% of the weight,
- infusion of mallow for approximately 25% of the weight,
- infusion of camomile for approximately 25% of the weight,
- infusion of sage for approximately 12.5% of the weight,
- infusion of thyme for approximately 6.25% of the weight,
- with the addition of lemon juice for approximately 6.25% of the weight.

7. Single dose pack according to one of Claims 5 or 6, in which the said substances (2) in the liquid state comprise a mixture of the following infusions:
- infusion of plantain for approximately 20% of the weight,
- infusion of mallow for approximately 20% of the weight,
- infusion of camomile for approximately 20% of the weight,
- infusion of sage for approximately 10% of the weight,
- infusion of dog rose with hibiscus flowers for approximately 10% of the weight,
- infusion of orange flowers for approximately 10% of the weight,
- infusion of thyme for approximately 5% of the weight,
- with the addition of lemon juice for approximately 5% of the weight.

8. Single dose pack according to one of claims from 5 to 7, in which a sweetener is added to the said substances (2) in the liquid state.

9. Single dose pack according to Claim 8, in which the said sweetener comprises honey.

10. Single dose pack according to one of claims from 5 to 9, in which the infusions comprising the said mixture contain water in which the following respectively have been infused:
- approximately 1.5% by weight of plantain,
- approximately 0.9% by weight of mallow,
- approximately 0.9% by weight of camomile,
- approximately 1.5% by weight of sage,
- approximately 5.4% by weight of dog rose with hibiscus flowers,
- approximately 2.4% by weight of orange flowers,
- approximately 1% by weight of thyme.

11. Container according to one of the preceding claims, in which the volume of the said cavity (3c) housing a single dose is approximately 30 cm³.
